# EUROPEAN PATENT APPLICATION

(11) **EP 2 571 036 A1**
(43) Date of publication of application: **20.03.2013**
(21) Application number: 11181547.8
(22) Date of filing: 16.09.2011
(51) Int. Cl.: H01H 3/14, G06F 3/033, A61B 17/00

(54) **Footswitch**

(71) Applicant: ARTHREX MEDIZINISCHE INSTRUMENTE GmbH, D-85757 Karlsfeld (DE)
(72) Inventor: Böhm, Michael, 85757 Karlsfeld (DE); Honisch, Michael, 85258 Ebersbach (DE)
(74) Representative: Lohr, Georg

(57) **Abstract**

A foot switch comprises of a first switch housing (10) which can be located on the floor and a U-shaped frame (30) comprising of two sidewalls (31,32) being attached to the sides of the switch housing. There is a second switch housing (20) attached to top of the frame. The switch housings contain a plurality of switching elements which can be actuated by preferably pressing them down. An operator's foot may be placed on the foot switch between the left side wall and the right side wall. Both walls limit the foot's movement and give a tactile feedback on the position. At least one or preferably multiple switching elements (12,15) are placed close to the walls (31,32). Therefore the switching elements may be unambiguously operated by moving the foot towards a sidewall and pressing the foot down.

## Description

### Field of the invention

The invention relates to an electrical foot switch. Such a switch may be used to control medical devices and instruments.

### Description of the related art

Foot switches are commonly used to control medical devices and instruments like lasers, RF surgery generators or drills. The main advantage is that the surgeon has the hands free for other instruments. Furthermore the requirements on sterility are lower as the switch can be kept on the floor. As the number of instruments in the operating room is increasing, the demand for controls also increases. This may lead to a large number of similar switches like the switch disclosed in EP 0 512 751 A2, being located under the operating table, leading to a dangerous configuration, as a confusion of switches may lead to unwanted functions or malfunctions of the attached instruments.

An approach to integrate a plurality of switching functions is disclosed in EP 1 217 640 A1. Here two or three switches are mechanically connected together to provide a defined spatial relationship. Anyway the number of available switches is low. US 6,051,797 discloses multiple switches in one common housing. The switches are provided at different positions at different levels. Therefore moving a foot from one switch to another may require looking under the operating table at the foot switch. This interrupts working flow and is therefore not desirable.

### Summary of the invention

The problem to be solved by the invention is to provide a foot switch which integrates a comparatively high number of switching elements and still allows a reliable location of the position of the individual switching elements.

Solutions of the problem are described in the independent claims. The dependent claims relate to further improvements of the invention.

The foot switch comprises of a first switch housing which can be located on the floor or on any support base. This switch housing contains a plurality of switching elements which can be actuated by preferably pressing them down. To give a tactile feedback of the foot location, at least one foot locator is provided. This foot locator can be touched by the foot and allows easy determining the position of the foot relative to the switch only by tactile sensation without viewing the switch. Preferably the foot locator is a post protruding from the surface of the switch. It may have a cylindrical shape. The foot switch has a notch at its front side to give further feedback of the foot location.

According to a further embodiment a frame is provided defining at least a right side wall and a left side wall. Both walls are connected on the top by a top section to form a rigid U-shaped frame. An operator's foot may be placed on the foot switch between the left side wall and the right side wall. Both walls limit the foot's movement and give a tactile feedback on the position. At least one or preferably multiple switching elements are placed close to the walls. Therefore the switching elements may be unambiguously operated by moving the foot towards a sidewall and pressing the foot down.

In a preferred embodiment the foot switch has a rear wall further limiting foot's movement to the rear, preferably in a direction at right angle to the side walls and giving tactile feedback into the direction of the rear side.

In a further embodiment a second switch housing is held by the top section. This allows the integration of additional switching elements into the foot switch.

In a further embodiment means for guiding cables or wires like a cable duct is provided within the left side wall or the right side wall.

An advantage of the invention is the flexibility and the modularity. Different foot switches can easily be combined to a complex assembly. Due to the bracket the spatial relationship between the switches remains constant. This ensures reliable orientation of the surgeon. Furthermore the sidewalls of the bracket give tactile feedback of the position without limiting mobility of the foot.

The invention has been described by means of an example of a foot switch for medical applications. It is obvious that such a foot switch also may be used for any other application where foot switches are required.

### Description of Drawings

In the following the invention will be described by way of example, without limitation of the general inventive concept, on examples of embodiment with reference to the drawings.
Figure 1 shows an embodiment of the foot switch in a side view.
Figure 2 shows the foot switch in a different view.
Figure 3 shows a further embodiment of the foot switch.
Figure 4 shows another view of the foot switch.

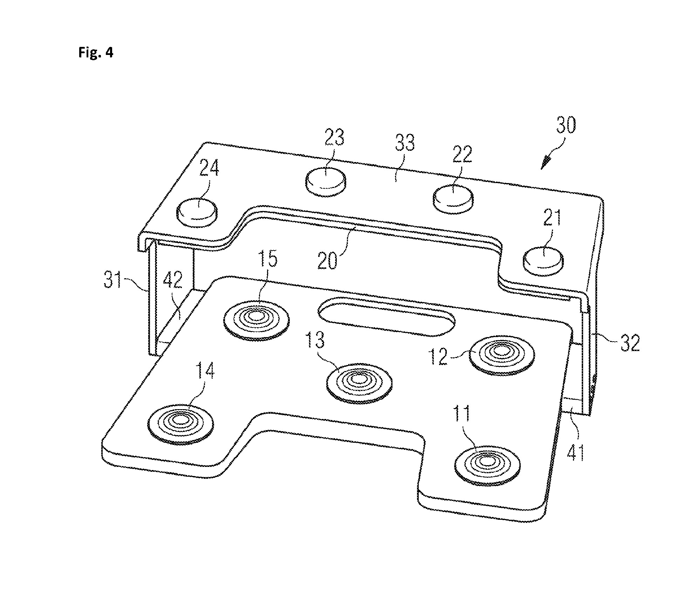

Figure 4 shows a different embodiment of the foot switch.

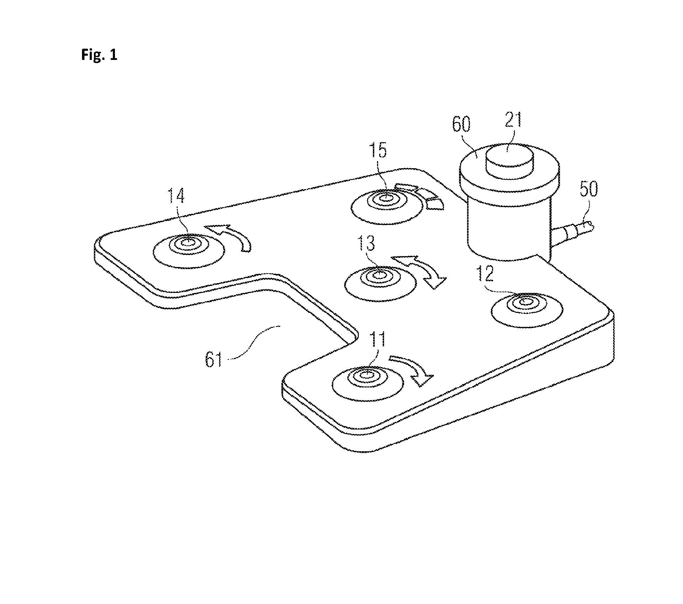

In figure 1 a preferred embodiment according to the invention is shown. Solutions of the problem are described in the independent claims. The dependent claims relate to further improvements of the invention.

The foot switch comprises of a first switch housing 10. The case may be located on the floor. It may have rubber feet to prevent slipping on the floor. The switch housing contains a plurality of foot switching elements 11-15. Preferably the switching elements are operated by pressing them downwards. The mechanical or electronic switching elements may have some rubber or plastic cover. At least a foot locator 60 is provided to give a tactile feedback on the position. Preferably the foot locator 60 bears at least one switch 21. Furthermore the switch has a notch at its front side which allows further location of the foot, specifically in relationship to the switching elements 11, 13 and 14. For connecting the switch a cable 50 may be provided.

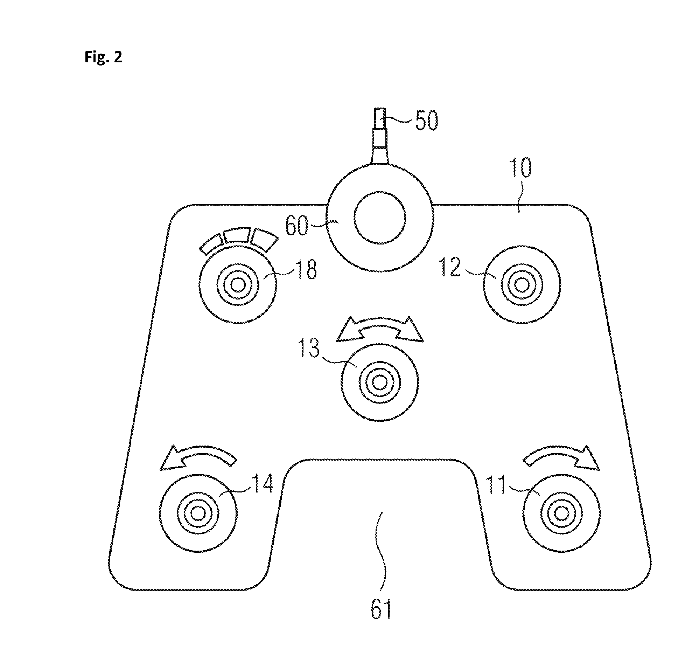

In figure 2 the foot switch is shown at a different view.

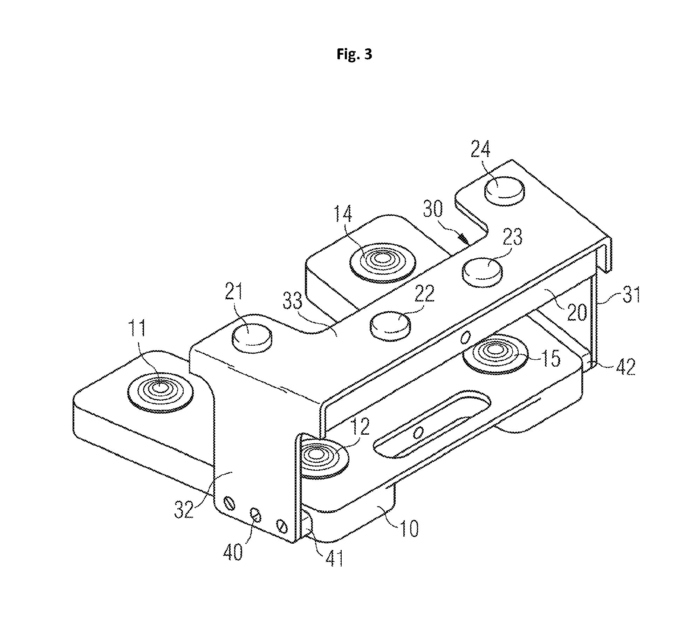

Figure 3 shows another embodiment of the foot switch. The foot switch comprises of a first switch housing 10. The case may be located on the floor. It may have rubber feet to prevent slipping on the floor. The switch housing contains a plurality of foot switching elements 11-15. Preferably the switching elements are operated by pressing them downwards. The mechanical or electronic switching elements may have some rubber or plastic cover. At least a right side wall 32 and a left side wall 31 are provided to limit a foot's movement and give a tactile feedback on the position. At least one switch 12 is located left of the right sidewall 32 in a position close to the right sidewall guiding a foot touching the right sidewall to the position of this switch. Furthermore at least one switch 15 is located right of the left side wall 31 in a position close to the left sidewall guiding the foot touching the left side wall to the position of this switch. The right sidewall 32 and the left sidewall 22 are connected by means of a cross bar 33 to further stabilize the sidewalls. The sidewalls and the crossbar form a frame 30. This frame may have means for guiding cables or wires between switch housings.

Furthermore a second switch housing 20 is attached to this cross bar. This switch housing contains switches 21, 22, 23 and 24. The sidewalls 31, 32 are fixed to the first switch housing 10 via spacers 41, 42 by means of screws 40.

Figure 4 shows the foot switch from a different view.

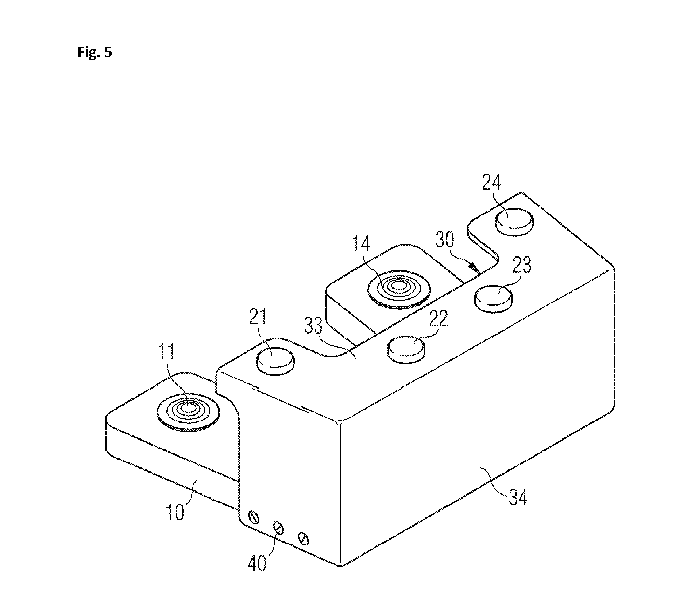

Figure 5 shows the foot switch with a rear wall 34. This wall guides the foot in the backward direction and allows a precise location of the foot. When the foot touches rear wall 34 and left sidewall 31, it is precisely located above switching element 15. When the foot touches rear wall 34 and right sidewall 32, it is precisely located above switching element 12.

### List of reference numerals

- 10: first switch housing
- 11 - 15: switching elements in first switch housing
- 20: second switch housing
- 21- 24: switching elements in second switch housing
- 30: frame
- 31: left side wall
- 32: right side wall
- 33: cross bar
- 34: rear wall
- 40: screws
- 41: right spacer
- 42: left spacer
- 50: cable
- 60: foot locator
- 61: notch

## Claims

1. Foot switch comprising of a switch housing (10), having at least two switching elements (11, 12, 13, 14, 15),
**characterized in, that**
a foot locator (60) is provided protruding from the surface of the switch housing (10), allowing a tactile feedback of a foot's position with respect to at least one of the switching elements by touching the foot locator.

2. Foot switch according to claim 1,
**characterized in, that**
the foot locator (60) has a cylindrical shape.

3. Foot switch according to claim 1 or 2,
**characterized in, that**
at least one switch element is located at the top of the foot locator (60).

4. Foot switch comprising of a first switch housing (10), having at least two switching elements (11, 12, 13, 14, 15),
**characterized in, that**
a left sidewall (31) is provided at a left side of the switch,
a right sidewall (32) is provided at the right side of the switch, and
at least one of the switching elements (15) is located at the right side of the left side wall and another of the switching elements (12) is located at the left side of the right sidewall so that the side walls act as limit stops for a foot defining the correct position of the foot above the switching elements.

5. Foot switch according to claim 4,
**characterized in, that**
a cross bar (33) is provided, connecting the left sidewall (31) and the right sidewall (32) to a frame (30).

6. Foot switch according to claim 5,
**characterized in, that**
a second switch housing (20) is attached to the crossbar (33).

7. Foot switch according to claim 4 or 5,
**characterized in, that**
a rear wall (34) is provided, acting as a limit stop for a foot in a rear direction.
